# FASCICULE DE BREVET EUROPEEN

(11) **EP 1 795 135 B1**
(45) Date de publication et mention de la délivrance du brevet: **04.03.2009**
(21) Numéro de dépôt: 06356137.7
(22) Date de dépôt: 06.12.2006
(51) Int. Cl.: A61B 17/70

(54) **Dispositif de stabilisation dynamique latérale du rachis**
Einrichtung zur lateralen dynamischen Stabilisierung der Wirbelsäule
Apparatus for lateral dynamic lateral stabilisation of the spine

(30) Priorité: 07.12.2005 FR 0512427
(43) Date de publication de la demande: 13.06.2007
(73) Titulaire: PHUSIS, 38330 Saint Ismier (FR)
(72) Inventeur: Ferrari épouse Gosset, Irène, 38660 Saint Vincent de Mercuze (FR); Tornier, Alain, 38330 St. Ismier (FR)
(74) Mandataire: Myon, Gérard Jean-Pierre

(56) Documents cités:
- DE-U1- 29 814 320
- US-A- 5 423 816
- US-A1- 2005 113 927
- US-B1- 6 296 644
- PATENT ABSTRACTS OF JAPAN vol. 1999, no. 01, 29 janvier 1999 (1999-01-29) & JP 10 277070 A (SANO SHIGEO; ITO KAORU), 20 octobre 1998 (1998-10-20)

## Description

La présente invention concerne un dispositif de stabilisation latérale du rachis, destiné à être implanté le long de la colonne vertébrale, au niveau d'un ou des deux de ses côtés latéraux gauche et droit, en vue de stabiliser au moins deux vertèbres l'une par rapport à l'autre. Une telle stabilisation est recherchée notamment dans le cadre du traitement du rachis dégénératif ou traumatique. L'invention s'intéresse plus particulièrement au traitement du rachis dorso-lombaire mais s'applique également au traitement du rachis cervical.

Pour traiter une instabilité intervertébrale, une première possibilité connue consiste à fusionner deux vertèbres adjacentes, ce qui revient à priver ces deux vertèbres de leur liberté de mouvements relatifs. Des montages totalement rigides sont à cet effet implantés de manière fixe le long du rachis pour bloquer définitivement la liaison articulaire entre les deux vertèbres à fusionner. US-B-6,296,644 propose ainsi un montage vertébral constitué de plusieurs éléments vertébraux qui sont chacun à fixer sur autant de vertèbres et qui sont reliés deux à deux par des liaisons « blocables » : lors de la mise en place du montage, ces liaisons sont mobiles, pour accommoder le positionnement relatif des éléments vertébraux le long du rachis, puis, à la fin de cette mise en place, les liaisons sont figées à demeure par des bagues à mémoire de forme, de sorte que, en service, les éléments vertébraux sont complètement fixes les uns par rapport aux autres. Ce genre d'intervention d'arthrodèse des vertèbres conduit cependant à une dégénérescence des disques adjacents sur lesquels il est nécessaire d'intervenir ultérieurement.

Une autre possibilité connue de traitement du rachis consiste à intervenir à un stade plus précoce que celui impliquant une arthrodèse. Une première solution de ce type est proposée dans DE-U-298 14 320 : plusieurs éléments vertébraux distincts, respectivement fixés à des vertèbres adjacentes, sont en service mobiles les uns par rapport aux autres, en étant reliés deux à deux par des liaisons télescopiques rectilignes suivant la direction longitudinale du rachis. Ce montage mobile s'accommode d'une certaine évolution du comportement cinématique du rachis, par exemple lors de sa croissance, mais n'assure pas une réelle stabilisation dynamique des vertèbres, n'empêchant ainsi pas, par exemple, l'écrasement ou la déformation des disques intervertébraux.

Une deuxième solution est proposée dans JP-A-10 277070 : deux pistons relient verticalement les côtés postérieurs de deux vertèbres adjacentes, avec interposition, entre la partie mâle et la partie femelle de chaque piston, d'une chemise en matériau élastique. Chaque piston présente une section transversale elliptique, ce qui, d'une part, empêche les parties mâle et femelle du piston d'être guidées en rotation l'une par rapport à l'autre autour d'un axe vertical et, d'autre part, centre les mouvements d'articulation entre ces parties mâle et femelle soit dans le piston, soit dans un plan passant par les deux pistons, c'est-à-dire bien en arrière des vertèbres. La cinématique imposée aux vertèbres est ainsi très éloignée des comportements anatomiques normaux du rachis, avec des risques importants que le disque intervertébral se retrouve pincé, voire écrasé au moins dans sa portion antérieure.

D'autre solutions visent à implanter un dispositif latéral de stabilisation dynamique, tel que ceux proposés dans US-A-5,423,816, US-A-5,704,936 et US-B-6,616,669. A cet effet, ce genre de dispositif comporte, d'une part, des éléments rigides à ancrer dans l'os d'un même côté latéral de deux vertèbres adjacentes et, d'autre part, des éléments souples de liaison entre ces éléments rigides. Ces éléments souples, tels que des ressorts ou des bras flexibles, s'étendent latéralement le long du rachis et soulagent ainsi le disque intervertébral en réduisant d'éventuelles surpressions au niveau des surfaces articulaires entre ce disque et les vertèbres. Ces dispositifs offrent plus de confort au patient car ils permettent de conserver la mobilité du rachis. Cependant, en pratique, l'utilisation de ce genre de dispositif dynamique se révèle délicate. Le dimensionnement de la flexibilité des éléments de liaison est difficile puisqu'il doit être adapté à chaque patient selon sa pathologie et sa morphologie et, à la longue, les comportements élastiques de ces éléments évoluent. Le mauvais contrôle de ces paramètres ne permet pas de garantir le respect d'une cinématique appropriée au rachis, ce qui peut conduire à une mauvaise stabilisation de l'écartement intervertébral et à une aggravation des lésions que l'on cherche à traiter.

Le but de la présente invention est de proposer un dispositif de stabilisation latérale du rachis reproduisant plus fidèlement les mouvements anatomiques des vertèbres, plus efficace pour stabiliser les vertèbres à traiter et plus fiable dans le temps.

A cet effet, l'invention a pour objet un dispositif de stabilisation latérale du rachis destiné, en service, à reproduire une liaison intervertébrale articulaire, tel que défini à la revendication 1.

Le fait de guider l'un par rapport à l'autre les deux éléments vertébraux du dispositif selon l'invention par les surfaces de guidage, délimitées par ces éléments et coopérant l'une avec l'autre par appui glissant, permet de conférer au dispositif un comportement cinématique précis et stable dans le temps. La cinématique imposée, à savoir un mouvement rotatif entre les deux éléments vertébraux autour du centre de rotation positionné, de manière prédéterminée, dans l'espace discal, garantit que les mouvements articulaires intervertébraux provoqués lors des sollicitations du rachis sont efficacement guidés pour être quasi-identiques ou, tout au moins, les plus proches possibles de comportements anatomiques normaux du rachis. De la sorte, la coopération de ces surfaces de guidage latérales peut permettre de conserver un espacement intervertébral satisfaisant, en maintenant un écartement vertical des vertèbres prédéterminé. Le dispositif selon l'invention supporte ainsi l'essentiel, voire la totalité des contraintes appliquées au disque intervertébral, ce dernier demeurant mobile. En outre, l'implantation du dispositif selon l'invention se révèle particulièrement facile : les mobilités internes au dispositif résident essentiellement, voire exclusivement, au niveau des surfaces de guidage portées par les deux éléments vertébraux dont les positions d'ancrage, de préférence latérales, dans les deux vertèbres à traiter sont choisies et fixées par le chirurgien. Comme ces surfaces de guidage s'étendent latéralement au rachis, les gestes chirurgicaux sont concentrés dans des zones latérales au rachis.

D'autres caractéristiques avantageuses de ce dispositif, prises isolément ou suivant toutes les combinaisons techniquement possibles, sont énoncées aux revendications dépendantes 2 à 11.

L'invention sera mieux comprise à la lecture de la description qui va suivre, donnée uniquement à titre d'exemple et faite en se référant aux dessins sur lesquels :
- la figure 1 est une vue schématique en perspective de deux vertèbres adjacentes équipées d'un dispositif de stabilisation latérale selon l'invention, ces vertèbres et ce dispositif étant observés par l'arrière, de manière décalée par rapport au plan sagittal du rachis et suivant une direction d'observation dirigée vers le bas ;
- la figure 2 est une section schématique selon le plan II de la figure 1 ; et
- la figure 3 est une vue en perspective et en éclaté d'une partie latérale du dispositif des figures précédentes.

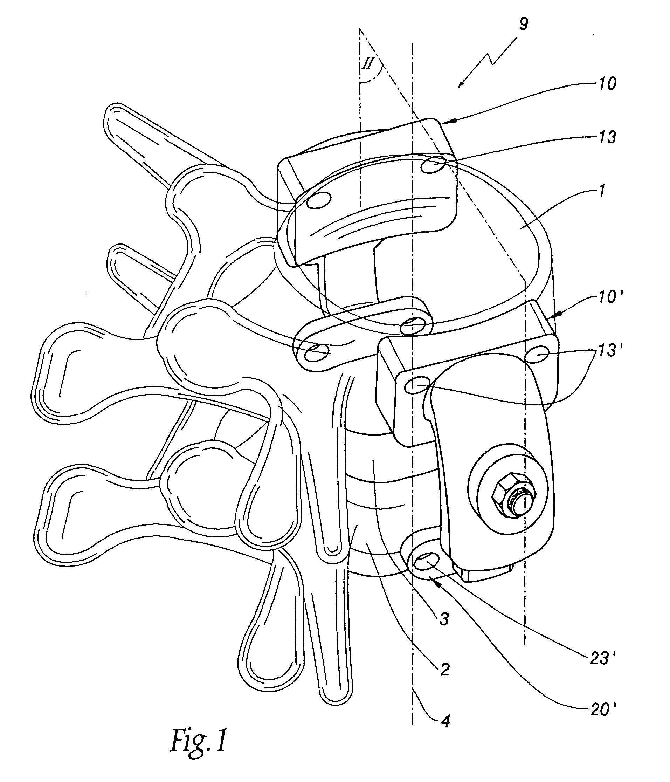

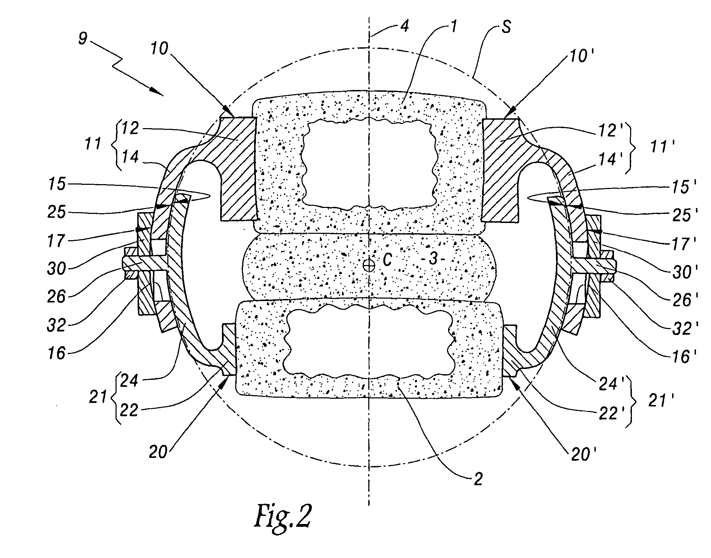

Sur les figures 1 et 2 sont représentées de manière schématique deux vertèbres adjacentes 1 et 2 d'un rachis lombaire d'un être humain, séparées par un disque intervertébral 3 suivant la direction longitudinale 4 de ce rachis. Par commodité, la suite de la description est orientée par rapport à ces vertèbres dans leur position anatomique, c'est-à-dire que les termes « postérieur » ou « arrière », « antérieur » ou « avant », « droit », « gauche », « supérieur », « inférieur », etc. s'entendent par rapport au rachis du patient se tenant debout. De même, le terme « sagittal » correspond à une direction dans le sens antéro-postérieur, verticalement sur la ligne médiane du rachis, tandis que le terme « médial » correspond à une direction sensiblement perpendiculaire au plan sagittal du rachis, dirigée vers ce rachis, le terme « latéral » correspondant à une direction de sens opposé.

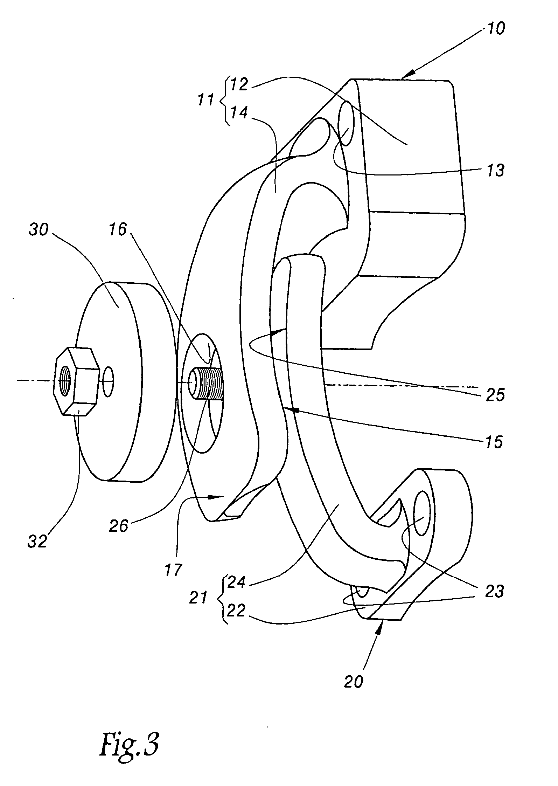

Sur les figures 1 à 3 est représenté un dispositif 9 de stabilisation dynamique des vertèbres 1 et 2, implanté sur les côtés gauche et droit des vertèbres, en vue de reproduire la liaison articulaire entre ces vertèbres, tout en assurant un espacement intervertébral satisfaisant. Ce dispositif comporte quatre éléments vertébraux, associés par paires prévues respectivement sur les côtés gauche et droit des vertèbres. La paire gauche inclut un élément vertébral supérieur 10 implanté au niveau de la vertèbre 1 et un élément vertébral inférieur 20 implanté au niveau de la vertèbre 2. De manière symétrique par rapport au plan sagittal du rachis, dont la trace dans le plan de la figure 2 correspond à la direction longitudinale 4 du rachis, la paire droite inclut deux éléments vertébraux 10' et 20' respectivement implantés au niveau des vertèbres 1 et 2. Par commodité, seuls les éléments vertébraux gauches seront décrits ci-après en détail, étant entendu que les éléments vertébraux droits 10' et 20' présentent des aménagements analogues, qui se déduisent par symétrie par rapport au plan sagittal du rachis et qui portent les mêmes références numériques que les aménagements des éléments gauches, mais suivies d'un prime.

Chaque élément vertébral 10, 20 comporte un corps rigide 11, 21 d'une seule pièce, notamment métallique, adapté pour être fixé au côté gauche de la vertèbre 1, 2. A cet effet, la partie supérieure 12 du corps 11 et la partie inférieure 22 du corps 21 sont traversées de part en part, suivant une direction médio-latérale, par des orifices 13, 23 destinés à recevoir des vis, non représentées, d'ancrage osseux dans le corps vertébral des vertèbres 1 et 2 pour immobiliser fermement les éléments vertébraux par rapport aux vertèbres.

La partie inférieure 14 de l'élément 10 et la partie supérieure 24 de l'élément 20 sont adaptées pour coopérer l'une avec l'autre lorsque le dispositif 9 est implanté, comme représenté sur les figures 1 et 2. Chacune de ces parties 14, 24 se présente globalement sous la forme d'une coupelle dont la concavité est tournée vers le côté gauche des corps des vertèbres 1 et 2. Les parties d'extrémité antérieure et postérieure de chacune de ces parties en coupelle 14 et 24 sont tronquées de sorte que la dimension principale de chaque partie 14 et 24 s'étend essentiellement le long du côté gauche des corps des vertèbres 1 et 2, c'est-à-dire sensiblement parallèlement à la direction longitudinale 4 du rachis. En coupe frontale, comme à la figure 2, c'est-à-dire dans un plan de coupe sensiblement vertical et parallèle à une direction médio-latérale, le corps 11, constitué des parties supérieure 12 et inférieure 14 venues de matière l'une avec l'autre, présente une section en forme globale de L retournée, dont la branche principale est bombée dans une direction opposée au rachis, tandis que le corps 21, constitué des parties inférieure 22 et supérieure 24 venues de matière l'une avec l'autre, présente une section en forme globale de L dont la branche principale est, elle aussi, bombée dans une direction opposée au rachis. Les parties 14 et 24 sont dimensionnées l'une par rapport à l'autre pour que, lorsque le dispositif 9 est implanté, la partie 14 recouvre latéralement la partie 24.

Plus précisément, la partie inférieure 14 de l'élément 10 délimite, sur sa face médiale, une surface concave 15, tandis que la partie supérieure 24 de l'élément 20 délimite, sur sa face latérale, une surface convexe 25 destinée à s'appuyer et à glisser contre la surface concave 15. Dans l'exemple considéré aux figures, ces surfaces 15 et 25 sont sensiblement complémentaires l'une de l'autre et correspondent approximativement à des portions d'une sphère S de centre C, comme représenté à la figure 2. Ainsi, lorsque les éléments 10 et 20 sont fixés aux vertèbres 1 et 2, les surfaces 15 et 25 sont appuyées l'une contre l'autre de manière glissante, et sont déplaçables l'une par rapport à l'autre par rotation autour du centre C, à la façon d'une liaison rotule de centre C.

En service, lorsque le dispositif 9 est implanté sur les vertèbres 1 et 2, les surfaces 15 et 25 coopèrent de manière à guider les mouvements articulaires entre les vertèbres autour du point C. Comme ce point est avantageusement situé dans l'espace discal intervertébral où est logé le disque 3, en particulier dans la région centrale de cet espace, les mouvements rotatifs imposés par la coopération de ces surfaces sont identiques ou, tout au moins, très proches des mouvements articulaires intervertébraux anatomiques générés lors d'une sollicitation du rachis. Le contact sphère/sphère entre les surfaces 15 et 25 apparente théoriquement le guidage réalisé à celui d'une rotule de centre C. En pratique, en raison des jeux entre ces surfaces et en raison de l'environnement ligamentaire des vertèbres 1 et 2, ces surfaces définissent, en coopérant, une pluralité de centres instantanés de rotation entre les éléments 10 et 20, tous situés avantageusement dans l'espace discal occupé par le disque 3. L'existence de cette pluralité de centres instantanés de rotation peut, en variante, être recherchée dans certains cas de traitements chirurgicaux, les surfaces 15 et 25 présentant alors des profils incurvés adaptés qui ne correspondent plus rigoureusement à des portions de la sphère S.

Par ailleurs, dans l'exemple considéré aux figures, les surfaces 15 et 25 des éléments gauches 10 et 20 d'une part, et les surfaces 15' et 25' des éléments droits 10' et 20' d'autre part, définissent des centres de rotation respectifs sensiblement confondus en C. Autrement dit, les surfaces 15' et 25' correspondent approximativement à des portions de la sphère S précitée, comme visible à la figure 2. En variante non représentée, les centres de rotation définis respectivement par les éléments gauches 10, 20 et droits 10', 20' peuvent ne pas être confondus, la cinématique du guidage imposé alors par le dispositif 9 étant plus complexe que celle décrite plus haut puisque les vertèbres 1 et 2 sont déplacées l'une par rapport à l'autre vis-à-vis de deux centres de rotation distincts, au moins instantanément. Ce genre de cinématique peut être recherché dans certains cas de traitements chirurgicaux du rachis.

Pour limiter l'amplitude des mouvements relatifs de rotation entre les éléments vertébraux 10 et 20, la partie 24 est munie d'un pion 26 venu de matière avec le reste de cette partie et s'étendant en saillie depuis la surface 25, dans une direction sensiblement médio-latérale dirigée à l'opposé du rachis. Ce pion 26 est reçu dans un orifice traversant 16 ménagé à travers la partie inférieure 14, qui débouche médialement sur la surface 15. Le diamètre du pion 26 est nettement inférieur au diamètre de l'orifice 16, de sorte que le pion peut s'y débattre librement, et ce dans toutes les directions ortho-radiales à la sphère S. Les mouvements rotatifs entre les éléments 10 et 20, autour du point C, sont autorisés jusqu'à ce que le pion 26 bute dans la paroi définissant l'orifice 16.

Pour renforcer le maintien juxtaposé des surfaces 15 et 25, une rondelle 30 est rapportée autour de l'extrémité libre du pion 26, en appui contre la surface latérale convexe 17 de la partie 14. Cette rondelle 30 est associée à un écrou de blocage 32, vissé autour de l'extrémité libre filetée du pion 26.

Des aménagements analogues, à savoir un pion 26', un orifice 16', une rondelle 30' et un écrou 32', sont prévus de manière symétrique au niveau des éléments vertébraux 10' et 20'.

L'implantation latérale du dispositif 9 est particulièrement aisée et rapide puisque seuls les éléments 10, 20, 10' et 20' sont à rapporter fermement aux vertèbres 1 et 2, au moyen des vis d'ancrage osseux précitées. En pratique, ces éléments sont mis en place simultanément, avec les parties inférieures 14 et 14' des éléments 10 et 10' recouvrant latéralement les parties supérieures 24 et 24' des éléments 20 et 20'. De la sorte, le chirurgien peut implanter le dispositif avec les surfaces 15 et 25 d'une part, et 15' et 25' d'autre part, au contact l'une de l'autre, pour une configuration d'extension prédéterminée des vertèbres. La coopération des surfaces 15 et 25 du côté gauche des vertèbres et la coopération des surfaces 15' et 25' du côté droit maintiennent en effet l'écartement longitudinal des vertèbres 1 et 2, suivant la direction 4, avec un écartement prédéterminé par le chirurgien.

Divers aménagements et variantes au dispositif 9 évoqué ci-dessus sont en outre envisageables :
- la structure de recouvrement des parties 14 et 24, 14' et 24' peut être inversée de sorte que, en variante non représentée, la partie supérieure de chaque élément vertébral inférieur recouvre latéralement la partie inférieure de chaque élément vertébral supérieur ;
- dans l'exemple de réalisation représenté aux figures, le dispositif 9 est implanté au niveau des côtés gauche et droit des vertèbres 1 et 2 ; en variante, ce dispositif peut ne comprendre qu'une seule paire, gauche ou droite, de deux éléments vertébraux, en particulier pour certains cas de traitement chirurgicaux spécifiques ; et/ou
- un même élément vertébral peut inclure à la fois une partie supérieure délimitant une première surface de guidage du type des surfaces 14, 24, 14' et 24', destinée à s'appuyer contre une surface associée d'un second élément vertébral situé au-dessus du premier élément, et une partie inférieure délimitant une seconde surface de guidage de ce type, destinée à s'appuyer contre une surface associée d'un troisième élément vertébral situé au-dessous du premier élément ; le dispositif correspondant s'étend alors le long de trois vertèbres adjacentes.

## Revendications

1. Dispositif (9) de stabilisation latérale du rachis destiné, en service, à reproduire une liaison intervertébrale articulaire, comportant au moins deux éléments vertébraux (10, 20, 10', 20') respectivement adaptés pour être fixés à un même côté latéral des corps d'au moins deux vertèbres adjacentes (1, 2), **caractérisé en ce que** les deux éléments vertébraux délimitent des surfaces respectives (15, 25, 15', 25') de guidage relatif des éléments, adaptées, lorsque les éléments sont implantés sur leur vertèbre correspondante, pour s'étendre globalement le long du même côté latéral du corps des vertèbres et pour s'appuyer et glisser l'une contre l'autre de telle sorte que ces surfaces définissent un centre de rotation (C), qui est situé dans l'espace discal intervertébral séparant les deux vertèbres adjacentes et autour duquel les deux éléments peuvent tourner l'un par rapport à l'autre.

2. Dispositif suivant la revendication 1, **caractérisé en ce que** les surfaces de guidage (15, 25, 15', 25') sont respectivement concave et convexe, en étant sensiblement complémentaires l'une de l'autre.

3. Dispositif suivant l'une quelconque des revendications précédentes, **caractérisé en ce que** chaque surface de guidage (15, 25, 15', 25') correspond sensiblement à une portion de sphère (S) dont le centre (C) correspond audit centre de rotation.

4. Dispositif suivant l'une quelconque des revendications précédentes, **caractérisé en ce que** les surfaces de guidage (15, 25, 15', 25') définissent un centre permanent de rotation (C) lorsque les vertèbres (1, 2) équipées des deux éléments vertébraux (10, 20, 10', 20') sont déplacées l'une par rapport à l'autre.

5. Dispositif suivant l'une quelconque des revendications 1 à 3, **caractérisé en ce que** les surfaces de guidage (15, 25, 15', 25') définissent plusieurs centres instantanés de rotation lorsque les vertèbres (1, 2) équipées des deux éléments vertébraux (10, 20, 10', 20') sont déplacées l'une par rapport à l'autre.

6. Dispositif suivant l'une quelconque des revendications précédentes, **caractérisé en ce qu'**il inclut des moyens (16, 26, 16', 26') de limitation de l'amplitude des mouvements relatifs de rotation entre les éléments vertébraux (10, 20, 10', 20').

7. Dispositif suivant la revendication 6, **caractérisé en ce que** les moyens de limitation comportent, d'une part, un pion (26, 26') solidaire de l'un (20, 20') des éléments vertébraux et s'étendant en saillie de la surface de guidage (25, 25') portée par cet élément, en direction de la surface de guidage (15, 15') portée par l'autre élément (10, 10'), et, d'autre part, un orifice (16, 16') adapté pour recevoir le pion (26) avec des jeux de débattement, cet orifice étant ménagé par l'autre élément vertébral (10, 10') et débouchant sur la surface de guidage (15, 15') que porte ce dernier.

8. Dispositif suivant l'une quelconque des revendications précédentes, **caractérisé en ce que** la partie (14, 24) de chaque élément vertébral (10, 20) sur laquelle est délimitée la surface de guidage correspondante (15, 25) se présente globalement sous la forme d'une coupelle tronquée, sensiblement centrée sur le centre de rotation (C).

9. Dispositif suivant la revendication 8, **caractérisé en ce que** chaque partie en forme de coupelle (14, 24) présente une concavité tournée vers le côté latéral des corps des vertèbres (1, 2) le long duquel les surfaces de guidage (15, 25) s'étendent.

10. Dispositif suivant l'une quelconque des revendications précédentes, **caractérisé en ce qu'**il comporte quatre éléments vertébraux (10, 20, 10', 20') associés par paires respectivement prévues pour être associées aux côtés gauche et droit des deux vertèbres (1, 2).

11. Dispositif suivant la revendication 10, **caractérisé en ce que** les centres de rotation respectivement associés à chaque paire d'éléments vertébraux (10, 20, 10', 20') sont sensiblement confondus.

## Claims

1. Device (9) for the lateral stabilisation of the spine, said device being intended to reproduce, when in use, an articular intervertebral link and comprising at least two vertebral elements (10, 20, 10', 20') which are respectively adapted to be fixed to one and the same lateral side of the bodies of at least two adjacent vertebrae (1, 2), **characterised in that** the two vertebral elements delimit respective surfaces (15, 25, 15', 25') for the relative guidance of said elements, which surfaces are adapted to extend, when the elements are implanted on their corresponding vertebra, totally along the same lateral side of the body of the vertebrae and to rest and slide against one another in such a way that the said surfaces define a centre of rotation (C) which is situated in the intervertebral discal space separating the two adjacent vertebrae and about which the two elements are capable of rotating in relation to one another.

2. Device according to claim 1, **characterised in that** the guide surfaces (15, 25, 15', 25') are respectively concave and convex, while being substantially complementary to one another.

3. Device according to any of the preceding claims, **characterised in that** each guide surface (15, 25, 15', 25') substantially corresponds to a portion of a sphere (S), the centre (C) of which corresponds to the said centre of rotation.

4. Device according to any of the preceding claims, **characterised in that** the guide surfaces (15, 25, 15', 25') define a permanent centre of rotation (C) when the vertebrae (1, 2) equipped with the two vertebral elements (10, 20, 10', 20') are displaced in relation to one another.

5. Device according to any of claims 1 to 3, **characterised in that** the guide surfaces (15, 25, 15', 25') define a number of instantaneous centres of rotation when the vertebrae (1, 2) equipped with the two vertebral elements (10, 20, 10', 20') are displaced in relation to one another.

6. Device according to any of the preceding claims, **characterised in that** it includes means (16, 26, 16', 26') for limiting the amplitude of the relative rotational movements between the vertebral elements (10, 20, 10', 20').

7. Device according to claim 6, **characterised in that** the limiting means comprise, on the one hand, a stud-bolt (26, 26') which is integral with one (20, 20') of the vertebral elements and extends, in a manner projecting from the guide surface (25, 25') carried by the said element, towards the guide surface (15, 15') carried by the other element (10, 10') and, on the other hand, an orifice (16, 16') which is adapted to receive the stud-bolt (26) with motion clearances, the said orifice being provided by the other vertebral element (10, 10') and opening onto the guide surface (15, 15') carried by the latter.

8. Device according to any of the preceding claims, **characterised in that** that part (14, 24) of each vertebral element (10, 20) on which the corresponding guide surface (15, 25) is delimited takes the form, as a whole, of a truncated cup which is substantially centred on the centre of rotation (C).

9. Device according to claim 8, **characterised in that** each cup-shaped part (14, 24) has a concavity which is turned towards that lateral side of the bodies of the vertebrae (1, 2) along which the guide surfaces (15, 25) extend.

10. Device according to any of the preceding claims, **characterised in that** it comprises four vertebral elements (10, 20, 10', 20') associated in pairs which are respectively intended to be associated with the left and right sides of the two vertebrae (1, 2).

11. Device according to claim 10, **characterised in that** the centres of rotation which are respectively associated with each pair of vertebral elements (10, 20, 10', 20') are substantially identical.

## Patentansprüche

1. Vorrichtung (9) zur seitlichen Stabilisierung der Wirbelsäule, die dazu bestimmt ist, nach ihrer Inbetriebnahme eine gelenkartige Verbindung zwischen den Wirbeln nachzubilden, und die mindestens zwei wirbelseitige Elemente (10, 20, 10', 20') aufweist, die jeweils dazu geeignet sind, auf derselben Seite der Körper mindestens zweier benachbarter Wirbel (1, 2) befestigt zu werden, **dadurch gekennzeichnet, dass** die zwei wirbelseitigen Elemente jeweils Flächen (15, 25, 15', 25') begrenzen, die der relativen Führung der Elemente dienen und derart ausgeführt sind, dass sie, nachdem die Elemente auf ihrem jeweiligen Wirbel implantiert wurden, sich insgesamt entlang derselben Seite des Körpers der Wirbel erstrecken und dass sie sich gegenseitig derart stützen und aneinander entlang gleiten, dass die Flächen einen Drehpunkt (C) festlegen, welcher im Bandscheibenbereich zwischen den beiden benachbarten Wirbeln liegt und um welchen die zwei Elemente gegeneinander Drehbewegungen ausführen können.

2. Vorrichtung nach Anspruch 1, **dadurch gekennzeichnet, dass** die Führungsflächen (15, 25, 15', 25') konkav beziehungsweise konvex geformt sind, wobei sie im Wesentlichen komplementär zueinander sind.

3. Vorrichtung nach einem beliebigen der vorhergehenden Ansprüche, **dadurch gekennzeichnet, dass** jede der Führungsflächen (15, 25, 15', 25') im Wesentlichen einem Abschnitt einer Kugel (S) entspricht, deren Mittelpunkt (C) dem Drehpunkt entspricht.

4. Vorrichtung nach einem beliebigen der vorhergehenden Ansprüche, **dadurch gekennzeichnet, dass** die Führungsflächen (15, 25, 15', 25') einen dauerhaften Drehpunkt (C) festlegen, wenn die Wirbel (1, 2), die mit den zwei wirbelseitigen Elementen (10, 20, 10', 20') versehen sind, gegeneinander bewegt werden.

5. Vorrichtung nach einem beliebigen der vorhergehenden Ansprüche 1 bis 3, **dadurch gekennzeichnet, dass** die Führungsflächen (15, 25, 15', 25') mehrere, zeitweilig wechselnde Drehpunkte festlegen, wenn die Wirbel (1, 2), die mit den zwei wirbelseitigen Elemente (10, 20, 10', 20') versehen sind, gegeneinander bewegt werden.

6. Vorrichtung nach einem beliebigen der vorhergehenden Ansprüche, **dadurch gekennzeichnet, dass** sie Mittel (16, 26, 16', 26') umfasst, die die maximale Auslenkung der Drehbewegungen begrenzen, welche die wirbelseitigen Elemente (10, 20, 10', 20') gegeneinander ausführen.

7. Vorrichtung nach Anspruch 6, **dadurch gekennzeichnet, dass** die Begrenzungsmittel einerseits einen Stift (26, 26') aufweisen, der mit einem (20, 20') der wirbelseitigen Elemente fest verbunden ist und sich ausgehend von der Führungsfläche (25, 25'), die von diesem Element getragen wird, in Richtung der Führungsfläche (15, 15') erstreckt, die von dem anderen Element (10, 10') getragen wird, sowie andererseits eine Öffnung (16, 16'), die dazu geeignet ist, den Stift (26) derart aufzunehmen, dass er Bewegungsspielraum in mehrere Richtungen hat, wobei die Öffnung in dem anderen wirbelseitigen Element (10, 10') vorgesehen ist und einen Bereich in der Führungsfläche (15, 15'), die das letztere trägt, freigibt.

8. Vorrichtung nach einem beliebigen der vorhergehenden Ansprüche, **dadurch gekennzeichnet, dass** der Abschnitt (14, 24) der jeweiligen wirbelseitigen Elemente (10, 20), welcher an die entsprechende Führungsfläche (15, 25) angrenzt, insgesamt die Form eines unvollständigen Schälchens aufweist, dessen Zentrum im Wesentlichen im Drehpunkt (C) liegt.

9. Vorrichtung nach Anspruch 8, **dadurch gekennzeichnet, dass** jeder der schälchenförmigen Abschnitte (14, 24) eine konkave Ausbuchtung aufweist, die zu derjenigen Seite der Körper der Wirbel (1, 2) zeigt, entlang welcher sich die Führungsflächen (15, 25) erstrecken.

10. Vorrichtung nach einem beliebigen der vorhergehenden Ansprüche, **dadurch gekennzeichnet, dass** sie vier wirbelseitige Elemente (10, 20, 10', 20') aufweist, die links und rechts der beiden Wirbel (1, 2) paarweise verbunden sind beziehungsweise dafür vorgesehen sind, dort verbunden zu werden.

11. Vorrichtung nach Anspruch 10, **dadurch gekennzeichnet, dass** die Drehpunkte, welche jeweils mit einem der beiden Paare wirbelseitiger Elemente (10, 20, 10', 20') in Verbindung stehen, im Wesentlichen zusammenfallen.
